# EUROPEAN PATENT APPLICATION

(11) **EP 4 450 059 A1**
(43) Date of publication of application: **23.10.2024**
(21) Application number: 22907748.2
(22) Date of filing: 22.11.2022
(51) Int. Cl.: A61K 8/9789, A61Q 13/00, A23L 33/105, A61K 36/535, A61K 36/534, A61P 43/00

(54) **ANTI-STRESS COMPOSITION COMPRISING NATURAL EXTRACT MIXTURE, AND USE THEREOF**

(30) Priority: 15.12.2021 KR 20210180023
(71) Applicant: Ajou University Industry-Academic Cooperation Foundation, Gyeonggi-do 16499 (KR)
(72) Inventor: JUNG, Yi-Sook, Yongin-si, Gyeonggi-do 16900 (KR); JEE, Hye Jin, Hwaseong-si, Gyeonggi-do 18378 (KR)
(74) Representative: Boult Wade Tennant LLP
(86) International application number: PCT/KR2022/018494
(87) International publication number: WO 2023/113278

(57) **Abstract**

The present invention relates to: an anti-stress composition comprising at least one extract selected from the group consisting of peppermint extract and perilla frutescens extract as a natural extract mixture; and a use thereof. Using peppermint extract and perilla frutescens extract in combination significantly alleviates stress and reduces cortisol levels as compared to using peppermint extract or perilla frutescens by itself, and thus a natural extract mixture in which peppermint extract and perilla frutescens extract are mixed together according to the present invention can be effectively used as an anti-stress composition, and furthermore, as a composition for preventing, ameliorating, or treating stress-related diseases, or in a method for preventing or treating stress-related diseases.

## Description

### [Technical Field]

This application claims the priority of Korean Patent Application No. 10-2021-0180023, filed on December 15, 2021, the entirety of which is a reference of the present application.

The present invention relates to an anti-stress composition including at least one extract selected from the group consisting of a *Mentha piperita* extract and a *Perilla frutescens var. acuta kudo* extract as a natural extract mixture, and a use thereof.

The present invention has been completed with the support of the Ministry of Science and ICT of Korea under Project No. 2020-JDH-3-CG-1 and Project No. 2021M3H1A1048922.

### [Background Art]

As society develops and diversifies rapidly, modern people are surrounded by various types of stress. Failure to successfully cope with stress results in psychological reactions such as depression, anxiety, fatigue, anger, and mood changes, as well as physiological reactions such as decreased α-waves in brain waves and increased blood pressure and pulse rate, but when this reaction continues to be repeated, diseases such as depression and anxiety appear to cause personal, family, and social losses, and lower the individual's quality of life. Recently, mental disorders in adolescents are also on the rise due to causes such as excessive desire to learn.

According to "Survey of Mental Disorders in Korea 2016" published by the Ministry of Health and Welfare, the '12-month prevalence rate of mental disorders', which is the proportion of the population experiencing one or more mental disorders in one year, is 12.1%, which is 1 of about 8 adults between the ages of 18 and 64 and 'the lifetime prevalence rate of mental disorders', which is the proportion of the population experiencing one or more mental disorders, is 26.6%, which is 1 of about 4 adults. The incidence rate is increasing worldwide, and the World Health Organization predicted that depression will become the most common disease among diseases at all ages in coming 2020. Nevertheless, only 18.2% of people diagnosed with mental disorders have any experience with counseling or treatment for mental problems.

Among mental disorders caused by stress, depression refers to a psychological state of depressed mood characterized by feelings of sadness, hopelessness, and frustration. The depression refers to a condition that progresses from the normal emotion of "gloomy" to major depressive disorder via dysthymic disorder. Symptoms of depression include major depressive episodes, which include changes in appetite and weight patterns, psychomotor agitation or retardation, decreased thinking ability, concentration or decision ability, lack of vitality, fatigue, worthlessness, remorse, or guilty conscience, frequent thoughts of death or suicide, plans or attempts to commit suicide, low mood in daily life, loss of interest or pleasure in most activities, etc.

There are various hypotheses about the biological cause of depression, but there is a prevailing view is that the biological cause is determined as disorders of the brain neurotransmitter system such as adrenaline, dopamine, or serotonin. Currently, tricyclic antidepressants (TCAs) or selective serotonin re-uptake inhibitors (SSRIs) are mainly used as treatments for depression, but the serotonin re-uptake inhibitors have the problem of being less effective and taking a long time. According to the American Psychiatric Association (APA) recommendations, the serotonin re-uptake inhibitor should be taken for at least 4 months, and may extend to 2 to 3 years depending on the patient. In addition, it is known that there is a risk that most depression symptoms will recur within 6 to 12 months after stopping the drug.

Most drugs used for mental disorders are classified as psychotropic drugs, and particularly, benzodiazepine drugs act directly on gamma-aminobutyric acid (GABA) receptors in the central nervous system, causing excessive sedation, and have a high risk of drug abuse due to high psychological or physical dependence.

### [Prior Art Documents]

### [Patent Documents]

(Patent Document 1) KR 10-2015-0063329 (May 6, 2015)

### [Disclosure]

### [Technical Problem]

The present inventors made intensive efforts to provide effective natural materials without side effects for stress or stress mental disorders, and thus confirmed that a *Perilla frutescens var. acuta kudo* extract and/or *Mentha piperita* extract was included to have an excellent stress-relieving effect and thus replace existing drugs used to treat stress or mental disorders, and then completed the present invention.

Therefore, an object of the present invention provides an anti-stress composition including at least one extract selected from the group consisting of a *Mentha piperita* extract and a *Perilla frutescens var. acuta kudo* extract as a natural extract mixture, and a use thereof.

### [Technical Solution]

One aspect of the present invention provides an anti-stress composition including at least one extract selected from the group consisting of *a Mentha piperita* extract and a *Perilla frutescens var. acuta kudo* extract.

According to a preferred embodiment of the present invention, the extract may be extracted from at least one part selected from the group consisting of leaves, stems, flowers, fruits, and whole plants of *Perilla frutescens var. acuta kudo* or *Mentha piperita.*

According to a preferred embodiment of the present invention, the extract may be extracted using water, an organic solvent, or a mixture thereof as a solvent.

According to a preferred embodiment of the present invention, the *Mentha piperita* extract and the *Perilla frutescens var. acuta kudo* extract may be mixed at a µg/ml concentration ratio of 1 : 10 to 10 : 1.

According to a preferred embodiment of the present invention, the composition may be a fragrance composition, a cosmetic composition, or a quasi-drug composition.

In addition, another aspect of the present invention provides a pharmaceutical composition for preventing or treating stress-related diseases including at least one extract selected from the group consisting of a *Mentha piperita* extract and a *Perilla frutescens var. acuta kudo* extract.

According to a preferred embodiment of the present invention, the stress-related disease may include at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

In addition, yet another aspect of the present invention provides a health functional food composition for preventing or ameliorating stress-related diseases including at least one extract selected from the group consisting of a *Mentha piperita* extract and a *Perilla frutescens var. acuta kudo* extract.

According to a preferred embodiment of the present invention, the stress-related disease may be at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

In addition, yet another aspect of the present invention provides a method for preventing or treating stress-related diseases including administering to a patient with stress-related diseases at least one extract selected from the group consisting of a *Mentha piperita* extract and a *Perilla frutescens var. acuta kudo* extract.

According to a preferred embodiment of the present invention, the stress-related disease may be at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

Yet another aspect of the present invention relates to an anti-stress composition including at least one extract selected from the group consisting of a *Mentha piperita* extract and a *Perilla frutescens var. acuta kudo* extract as a natural extract mixture, and a use thereof.

Specifically, when the composition of the present invention was treated in a sleep deprivation stress mouse model, it was confirmed that an immobility time was significantly reduced in a tail suspension test or forced swimming test (FIGS. 1 to 3c), and cortisol levels were significantly reduced (FIGS. 4 and 5). In addition, the anti-stress effect varied depending on a mixed µg/ml concentration ratio of the *Mentha piperita* extract and the *Perilla frutescens var. acuta kudo* extract, and it could be derived that an optimal mixed µg/ml concentration ratio thereof was 1 : 5, 3 : 1 or 4 : 6 (FIGS. 6 to 8).

Accordingly, yet another aspect of the present invention provides an anti-stress composition including at least one extract selected from the group consisting of a *Mentha piperita* extract and a *Perilla frutescens var. acuta kudo* extract.

According to a preferred embodiment of the present invention, the extract may be extracted from at least one part selected from the group consisting of leaves, stems, flowers, fruits, and whole plants of *Mentha piperita* or *Perilla frutescens var. acuta kudo.*

According to a preferred embodiment of the present invention, the extract may be extracted using water, an organic solvent, or a mixture thereof as a solvent.

According to a preferred embodiment of the present invention, the *Mentha piperita* extract and the *Perilla frutescens var. acuta kudo* extract may be mixed at a µg/ml concentration ratio of 1 : 10 to 10 : 1.

According to a preferred embodiment of the present invention, the composition may be a fragrance composition, a cosmetic composition, or a quasi-drug composition.

The fragrance may refer to all materials used to give off a scent, such as aroma. The fragrance composition may be included without limitation in any product that may be used for giving off the scent while exhibiting an anti-stress effect due to the *Mentha piperita* extract and/or the *Perilla frutescens var. acuta kudo* extract, but preferably included in at least one product selected from the group consisting of perfumes, scented candles, oils, air fresheners, detergents, and external skin preparations. The external skin preparations include all products necessary for bathing, such as soap, cleansers, or bath additives, but are not limited thereto.

The cosmetic composition of the present invention contains the *Mentha piperita* extract and/or the *Perilla frutescens var. acuta kudo* extract as an active ingredient, and may be prepared in the form of a basic cosmetic composition (lotion, skin protectant, cream, essence, cleanser such as cleansing foam and cleansing water, packs, body oil, and body cleanser), a colored cosmetic composition (foundation, lipstick, mascara, and makeup base), a hair product composition (shampoo, conditioner, scalp cleanser, hair conditioner, and hair gel), soap, etc., in addition to dermatologically acceptable excipients.

The excipient is not limited thereto, but may include, for example, a skin smoothing agent, a skin penetration enhancer, a colorant, an air fresher, an emulsifier, a thickener, and a solvent. In addition, flavorings, pigments, disinfectants, antioxidants, preservatives and moisturizing agents may be additionally included, and thickeners, inorganic salts, synthetic polymer materials, and the like may be included for the purpose of improving physical properties. For example, in the case of preparing the cleanser and the soap with the cosmetic composition of the present invention, the cleanser and the soap may be easily prepared by adding the *Mentha piperita* extract and/or the *Perilla frutescens var. acuta kudo* extract to general cleanser and soap base. In the case of preparing the cream, the cream may be prepared by adding the *Mentha piperita* extract and/or the *Perilla frutescens var. acuta kudo* extract or salts thereof to a conventional oil-in-water (O/W) type cream base. In addition, synthetic or natural materials such as proteins, minerals, vitamins, etc. may be further added thereto for the purpose of improving physical properties, in addition to fragrances, chelating agents, pigments, antioxidants, preservatives, etc.

The quasi-drug composition of the present invention refers to products with a milder action than drugs among products used for the purpose of diagnosing, treating, ameliorating, mitigating, handling or preventing human or animal diseases. For example, according to the Pharmaceutical Affairs Act, the quasi-drug includes products used for the treatment or prevention of human/animal diseases, products with mild or no direct action on the human body, etc., by excluding products used for the purpose of drugs.

When the *Mentha piperita* extract and/or the *Perilla frutescens var. acuta kudo* extract of the present invention are used as the quasi-drug composition, the composition may be added as it is or used together with other quasi-drug ingredients, and may be used appropriately according to conventional methods. The mixing amount of the active ingredients may be suitably determined according to the purpose of use (prevention, health, or therapeutic treatment).

Further, the present invention provides a pharmaceutical composition for preventing or treating stress-related diseases including at least one extract selected from the group consisting of a *Mentha piperita* extract and a *Perilla frutescens var. acuta kudo* extract.

Since the *Mentha piperita* extract and the *Perilla frutescens var. acuta kudo* extract are the same as those included in the anti-stress composition, the description thereof will be replaced by the above description.

According to a preferred embodiment of the present invention, the stress-related disease may be at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

The 'prevention' of the present invention means all actions that suppress or delay the onset of stress-related diseases or related diseases due to the *Mentha piperita* extract and/or the *Perilla frutescens var. acuta kudo* extract.

The 'amelioration' or 'treatment' of the present invention means all actions that improve or benefit parameters related to stress-related diseases or related diseases, such as the severity of symptoms due to the *Mentha piperita* extract and/or the *Perilla frutescens var. acuta kudo* extract.

The pharmaceutical composition of the present invention may be various oral or parenteral formulations. When the composition is formulated, the formulations may be prepared by using one or more buffers (e.g., saline or PBS), antioxidants, bacteriostatic agents, chelating agents (e.g., EDTA or glutathione), fillers, extenders, binders, adjuvants (e.g., aluminum hydroxide), suspending agents, thickening agents, wetting agents, disintegrants or surfactants, and diluents or excipients.

Solid formulations for oral administration include tablets, pills, powders, granules, capsules, etc., and these solid formulations may be prepared by mixing at least one excipient, for example, starch (including corn starch, wheat starch, rice starch, potato starch, etc.), calcium carbonate, sucrose, lactose, dextrose, sorbitol, mannitol, xylitol, erythritol maltitol, cellulose, methyl cellulose, sodium carboxymethyl cellulose and hydroxypropyl methyl cellulose or gelatin with one or more compounds. For example, tablets or sugar-coated tablets may be obtained by mixing an active ingredient with a solid excipient, pulverizing the mixture, adding a suitable auxiliary, and then processing the mixture into a granule mixture.

Further, lubricants such as magnesium stearate, talc, and the like may be used in addition to simple excipients. Liquid formulations for oral administration may correspond to suspensions, oral liquids, emulsions, syrups, and the like, and may include various excipients, such as a wetting agent, a sweetener, an aromatic agent, a preserving agent, and the like, in addition to water and liquid paraffin which are commonly used as simple diluents. In addition, in some cases, cross-linked polyvinylpyrrolidone, agar, alginic acid, sodium alginate, or the like may be added as a disintegrant, and anti-coagulants, fragrances, emulsifiers, solubilizers, dispersants, flavoring agents, antioxidants, packaging agents, pigments, preservatives, and the like may be additionally included.

Formulations for parenteral administration include a sterile aqueous solution, a non-aqueous solution, a suspension, an emulsion, a lyophilizing agent, a suppository, or the like. As the non-aqueous solution and the suspension, propylene glycol, polyethylene glycol, vegetable oil such as olive oil, injectable ester such as ethyl oleate, and the like may be used. As a base of the suppository, witepsol, macrogol, Tween 61, cacao butter, laurinum, glycerol, gelatin, and the like may be used.

The composition of the present invention may be administered orally or parenterally, and when administered parenterally, the composition may be formulated in the form of external skin use; an injection; a transdermal agent; or a nasal inhalant according to methods known in the art.

The injections need to be sterilized and protected from contamination of microorganisms such as bacteria and fungi. Examples of suitable carriers for injections may include, but are not limited to, solvents or dispersion media containing water, ethanol, polyols (e.g., glycerol, propylene glycol, and liquid polyethylene glycols), mixtures thereof and/or vegetable oils. More preferably, as suitable carriers, a Hanks' solution, a Ringer's solution, a phosphate buffered saline (PBS) containing triethanolamine or sterile water for injection, an isotonic solution such as 10% ethanol, 40% propylene glycol and 5% dextrose, and the like may be used. In order to protect the injection from microbial contamination, various antibacterial and antifungal agents such as paraben, chlorobutanol, phenol, sorbic acid, thimerosal, and the like may be further included. In addition, most of the injections may further include an isotonic agent, such as sugar or sodium chloride.

The transdermal agents are included in the form of ointments, creams, lotions, gels, external liquids, pastas, liniments, and aerosols. Hereinabove, the transdermal administration means that an effective amount of active ingredient contained in the pharmaceutical composition is delivered into the skin by topically applying the pharmaceutical composition to the skin.

In the case of the inhalants, the compound used according to the present invention may be conveniently delivered in the form of an aerosol spray from a pressurized pack or nebulizer by using a suitable propellant, for example, dichlorofluoromethane, trichlorofluoromethane, dichlorotetrafluoroethane, carbon dioxide, or other suitable gases. In the case of the pressurized aerosol, a dosage unit may be determined by providing a valve that delivers a metered amount. For example, gelatin capsules and cartridges used in an inhaler or insufflator may be formulated to contain a powder mixture of a compound and a suitable powder base such as lactose or starch.

The composition of the present invention is administered in a pharmaceutically effective amount. The pharmaceutically effective amount refers to an amount enough to treat the diseases at a reasonable benefit/risk ratio applicable to medical treatment. An effective dose level may be determined according to factors including the type and severity of a disease of a patient, the activity of a drug, the sensitivity to a drug, a time of administration, a route of administration, an excretion rate, duration of treatment, and simultaneously used drugs, and other factors well-known in the medical field. The composition of the present invention may be administered as an individual therapeutic agent or in combination with other therapeutic agents, and may be administered sequentially or simultaneously with conventional therapeutic agents, and administered singly or multiply. That is, the total effective amount of the composition of the present invention may be administered to a patient in a single dose, or may be administered according to a fractionated treatment protocol, which is administered in a multiple dose for a long period of time. It is important to administer an amount capable of obtaining a maximum effect with a minimal amount without side effects by considering all the factors and this may be easily determined by those skilled in the art.

The dose of the pharmaceutical composition of the present invention varies in the range thereof according to the weight, age, sex, health condition, diet, administration time, administration method, and excretion rate of a patient, the severity of disease, etc. The dose may increase or decrease depending on the route of administration, severity of obesity, sex, weight, age, etc.

The composition of the present invention may be used alone or in combination with surgery, radiation therapy, hormone therapy, chemotherapy, and methods of using biological response modifiers.

The pharmaceutical composition of the present invention may also be provided in the form of an external preparation including a *Mentha piperita* extract and/or the *Perilla frutescens var. acuta kudo* extract as an active ingredient. When the pharmaceutical composition for preventing or treating stress-related diseases of the present invention is used as an external skin preparation, the pharmaceutical composition may further contain adjuvants commonly used in the field of dermatology, such as any other ingredients commonly used in external skin preparations, such as fatty substances, organic solvents, solubilizers, thickening and gelling agents, emollients, antioxidants, suspending agents, stabilizers, foaming agents, air fresheners, surfactants, water, ionic emulsifiers, nonionic emulsifiers, fillers, sequestering agents, chelating agents, preservatives, vitamins, blockers, wetting agents, essential oils, dyes, pigments, hydrophilic activators, lipophilic activators or lipid vesicles. In addition, the ingredients may be introduced in an amount generally used in the field of dermatology.

When the pharmaceutical composition for preventing or treating stress-related diseases of the present invention is provided as an external skin preparation, the external skin preparation may be a formulation such as an ointment, a patch, gel, cream, or spray, but is not limited thereto.

Further, the present invention provides a health functional food composition for preventing or ameliorating stress-related diseases including at least one extract selected from the group consisting of *a Mentha piperita* extract and a *Perilla frutescens var. acuta kudo* extract.

Since the *Mentha piperita* extract and the *Perilla frutescens var. acuta kudo* extract are the same as those included in the anti-stress composition, the description thereof will be replaced by the above description.

According to a preferred embodiment of the present invention, the stress-related disease may be at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

The health functional food composition according to the present invention may be prepared in various forms according to general methods known in the art. General foods are not limited thereto, but may be prepared by adding the *Mentha piperita* extract and/or the *Perilla frutescens var. acuta kudo* extract of the present invention to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc. In addition, the nutritional supplement is not limited thereto, but may be prepared by adding the *Mentha piperita* extract and/or the *Perilla frutescens var. acuta kudo* extract of the present invention to capsules, tablets, pills, etc. In addition, the health functional food is not limited thereto, but for example, may be taken by liquefying, granulating, encapsulating, and powdering so as to be drunken (health beverages) by preparing the *Mentha piperita* extract and/or the *Perilla frutescens var. acuta kudo* extract itself in the form of tea, juice, and drinks. In addition, in order to use the *Mentha piperita* extract and/or the *Perilla frutescens var. acuta kudo* extract of the present invention in the form of a food additive, the *Mentha piperita* extract and/or the *Perilla frutescens var. acuta kudo* extract may be prepared and used in the form of powder or concentrate. In addition, the *Mentha piperita* extract and/or the *Perilla frutescens var. acuta kudo* extract of the present invention are mixed with active ingredients known to have a preventive or ameliorating effect on stress-related diseases to be prepared in the form of a composition.

When using the *Mentha piperita* extract and/or the *Perilla frutescens var. acuta kudo* extract of the present invention as a health drink, the health drink composition may contain various flavoring agents or natural carbohydrates as additional ingredients like conventional drinks. The natural carbohydrates described above may be monosaccharides such as glucose and fructose; disaccharides such as maltose and sucrose; polysaccharides such as dextrin and cyclodextrin; and sugar alcohols such as xylitol, sorbitol, and erythritol. As the sweetening agent, natural sweetening agents such as thaumatin and stevia extracts; synthetic sweetening agents such as saccharin and aspartame, and the like may be used. A ratio of the natural carbohydrates may be generally about 0.01 to 0.04 g, preferably about 0.02 to 0.03 g per 100 mL of the composition of the present invention.

In addition, the health functional food of the present invention may contain various nutrients, vitamins, electrolytes, flavoring agents, coloring agents, pectic acid and salts thereof, alginic acid and salts thereof, organic acid, protective colloidal thickeners, pH adjusting agents, stabilizers, preservatives, glycerin, alcohol, carbonic acid agents, or the like. In addition, the health functional food of the present invention may contain natural fruit juice, and pulp for preparing fruit juice beverage or vegetable beverage. These ingredients may be used independently or in combination.

In addition, the present invention may provide a method for preventing or treating stress-related diseases including administering to a patient with stress-related diseases at least one extract selected from the group consisting of a *Mentha piperita* extract and a *Perilla frutescens var. acuta kudo* extract.

Since the *Mentha piperita* extract and the *Perilla frutescens var. acuta kudo* extract are the same as those included in the anti-stress composition, the description thereof will be replaced by the above description.

According to a preferred embodiment of the present invention, the stress-related disease may be at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

### [Advantageous Effects]

According to the present invention, using *Mentha piperita* extract and *Perilla frutescens var. acuta kudo* extract in combination significantly alleviates stress and reduces cortisol levels as compared to using *Mentha piperita* extract or *Perilla frutescens var. acuta kudo* extract by itself, and thus the natural extract mixture in which *Mentha piperita* extract and *Perilla frutescens var. acuta kudo* extract are mixed together according to the present invention can be effectively used as an anti-stress composition, and furthermore, as a composition for preventing, ameliorating, or treating stress-related diseases, or in a method for preventing or treating stress-related diseases.

### [Description of Drawings]

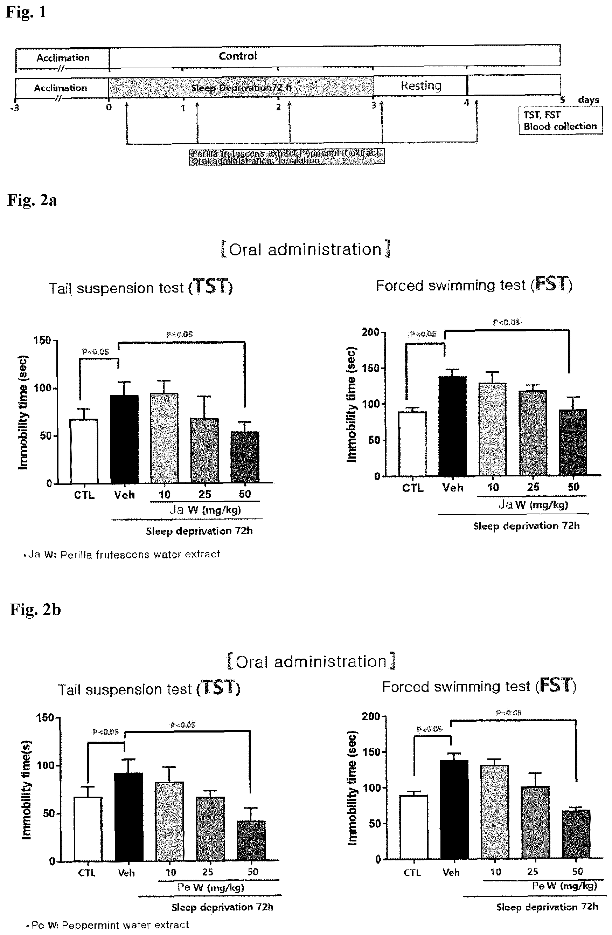

FIG. 1 shows a series of experimental methods for administering a natural extract mixture to a sleep deprivation stress mouse model prepared in the present invention.
FIG. 2A shows results of confirming a stress ameliorating effect when a *Perilla frutescens var. acuta kudo* extract (Ja W) of the present invention is orally administered to a sleep deprivation stress mouse model using a tail suspension test and a forced swimming test. It was confirmed that when mixing the natural product extracts, a synergy occurred in the stress ameliorating effect.
FIG. 2B shows results of confirming a stress ameliorating effect when a *Mentha piperita* extract (Pe W) of the present invention is orally administered to a sleep deprivation stress mouse model using a tail suspension test and a forced swimming test. It was confirmed that when mixing the natural product extracts, a synergy occurred in the stress ameliorating effect.

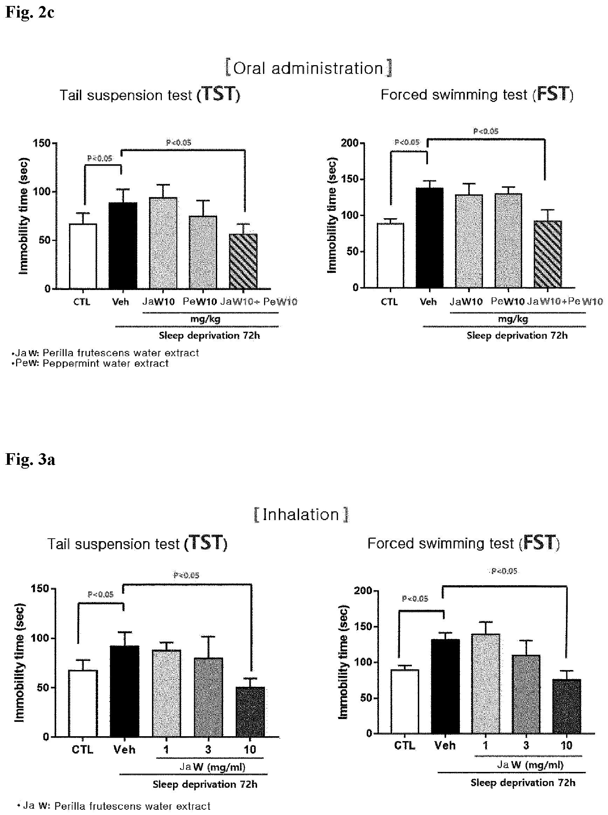

FIG. 2C shows results of confirming a stress ameliorating effect when natural product extracts (Ja W, Pe W, and Ja W + Pe W) of the present invention are orally administered to a sleep deprivation stress mouse model using a tail suspension test and a forced swimming test. It was confirmed that when mixing the natural product extracts, a synergy occurred in the stress ameliorating effect.
FIG. 3A shows results of confirming a stress ameliorating effect when a *Perilla frutescens var. acuta kudo* extract (Ja W) of the present invention is inhaled to a sleep deprivation stress mouse model using a tail suspension test and a forced swimming test. It was confirmed that when mixing the natural product extracts, a synergy occurred in the stress ameliorating effect.

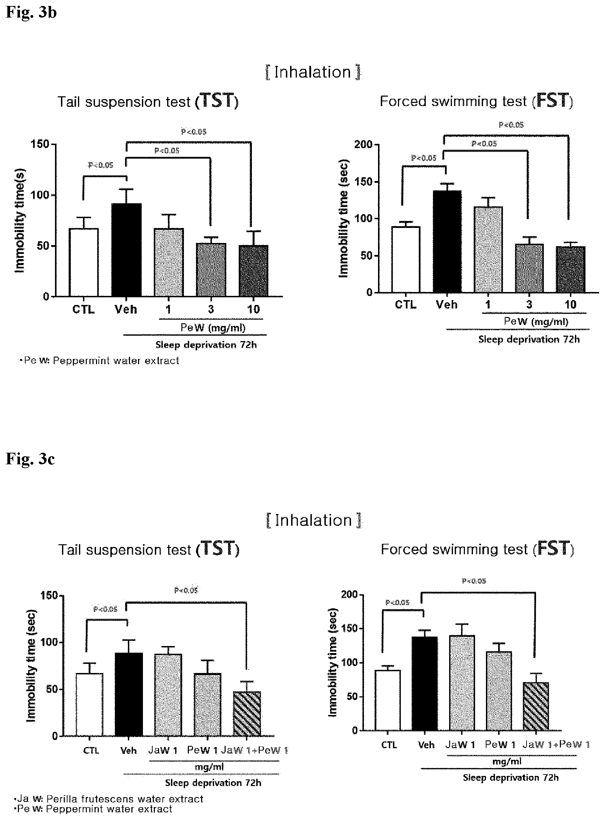

FIG. 3B shows results of confirming a stress ameliorating effect when a *Mentha piperita* extract (Pe W) of the present invention is inhaled to a sleep deprivation stress mouse model using a tail suspension test and a forced swimming test. It was confirmed that when mixing the natural product extracts, a synergy occurred in the stress ameliorating effect.
FIG. 3C shows results of confirming a stress ameliorating effect when natural product extracts (Ja W, Pe W, and Ja W + Pe W) of the present invention are inhaled to a sleep deprivation stress mouse model using a tail suspension test and a forced swimming test. It was confirmed that when mixing the natural product extracts, a synergy occurred in the stress ameliorating effect.

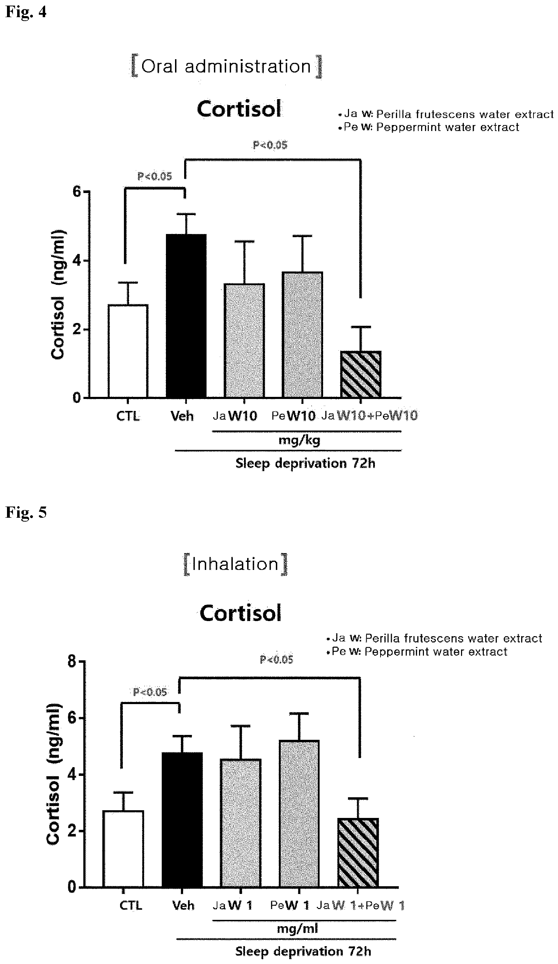

FIG. 4 shows a cortisol reducing effect when natural product extracts (Ja W, Pe W, and Ja W + Pe W) of the present invention are orally administered to a sleep deprivation stress mouse model. It was confirmed that when mixing the natural product extracts, a synergy occurred in the cortisol reducing effect.
FIG. 5 shows a cortisol reducing effect when natural product extracts (Ja W, Pe W, and Ja W + Pe W) of the present invention are inhaled to a sleep deprivation stress mouse model. It was confirmed that when mixing the natural product extracts, a synergy occurred in the cortisol reducing effect.

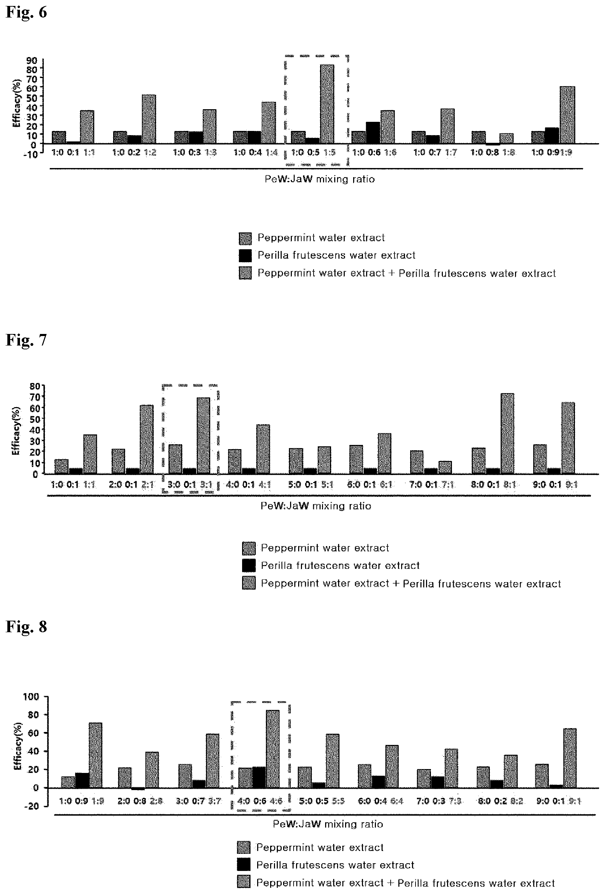

FIG. 6 shows an anti-stress effect according to a mixing ratio of each natural product extract when natural product extracts (Ja W, Pe W, and Ja W + Pe W) of the present invention are treated to nerve cells. It was confirmed that the anti-stress effect significantly increased when the mixed µg/ml concentration ratio of a *Mentha piperita* water extract and a *Perilla frutescens var. acuta kudo* water extract was 1 : 5.
FIG. 7 shows an anti-stress effect according to a mixing ratio of each natural product extract when natural product extracts (Ja W, Pe W, and Ja W + Pe W) of the present invention are treated to nerve cells. It was confirmed that the anti-stress effect significantly increased when the mixed µg/ml concentration ratio of a *Mentha piperita* water extract and a *Perilla frutescens var. acuta kudo* water extract was 3 : 1.
FIG. 8 shows an anti-stress effect according to a mixing ratio of each natural product extract when natural product extracts (Ja W, Pe W, and Ja W + Pe W) of the present invention are treated to nerve cells. It was confirmed that the anti-stress effect significantly increased when the mixed µg/ml concentration ratio of a *Mentha piperita* water extract and a *Perilla frutescens var. acuta kudo* water extract was 4 : 6.

### [Best Mode of the Invention]

### [Example 1]

### Preparation of natural product extract

*Perilla frutescens var. acuta kudo* or *Mentha piperita* were hot-water extracted at 80°C and then sterilized at 121 °C for 20 minutes to prepare a *Perilla frutescens var. acuta kudo* or *Mentha piperita* hot-water extract (Ja W, Pe W).

### [Example 2]

### Preparation of sleep deprivation stress mouse model

After water was filled in a cage where a platform was erected, an ICR mouse (Orient Bio Co., Ltd.) was placed on the platform and then sleep deprived for 3 days. At this time, the top of the animal cage equipped with a filter was attached with a gauze surface with a size of 5 cm × 5 cm in which 5 ml of 0.9% physiological saline (CTL, Veh) and 1 mg/ml of the natural product extract prepared in [Example 1] (Ja W and/or Pe W) were sufficiently absorbed for 5 days, and installed so that the smell spread inside the cage. Alternatively, the natural product extract (Ja W and/or Pe W) was administered orally at 10 mg/kg. After the stress induction was completed, the mice were stabilized for 24 hours, and then subjected to a tail suspension test (TST) or a forced swimming test (FST) to determine the immobility time of each mouse.

### [Example 3]

### Confirmation of effect of natural product extracts

### <3-1> Tail suspension test

After stress was induced in [Example 2], a tape was attached to the end of the stabilized mouse's tail and the mouse was hung upside down on the wall. Movement was observed for a total of 6 minutes, and the levels of stress, etc., were measured by measuring the time to remain motionless and immobile.

As a result, the immobility time of mice induced by stress and the like due to sleep deprivation significantly increased (Veh). In addition, it was confirmed that when the mice were treated with the *Perilla frutescens var. acuta kudo* extract (Ja W) and/or *Mentha piperita* extract (Pe W), the effect of reducing immobility time significantly increased (FIGS. 2A to 3C).

### <3-2> Forced swimming test

After filling a water tank of about 30 cm with water, mice stabilized after inducing the stress in [Example 2] above were placed therein. Movement was observed for a total of 6 minutes, and the levels of stress, etc., were measured by measuring the time to remain motionless and immobile.

As a result, the immobility time of mice induced by stress and the like due to sleep deprivation significantly increased (Veh). In addition, it was confirmed that when the mice were treated with the *Perilla frutescens var. acuta kudo* extract (Ja W) and/or *Mentha piperita* extract (Pe W), the effect of reducing immobility time significantly increased (FIGS. 2A to 3C).

### [Example 4]

### Confirmation of cortisol levels

Ja W and/or Pe W was administered orally or inhaled to mice with sleep deprivation stress 72 h-induced depression in [Example 1], and then the plasma was separated from the mouse's blood, and the concentration of cortisol was measured using an ELISA kit Cortisol Assay Kit (MyBioSource, USA).

Specifically, in a built-in 96-well microplate, 50 µl of a built-in cortisol standard solution was dispensed for each concentration to create a standard curve, and 50 µl of the mouse plasma was dispensed into new wells, and 50 µl of a cortisol conjugate was added to each well. Then, 50 µl of a primary antibody solution was added and the 96-well microplate was reacted at 37°C for 1 hour. Thereafter, the microplate was washed three times with a washing solution, and 100 µl of a substrate solution was added to each well and reacted at 37°C for 15 minutes. After the reaction, 50 µl of a reaction stop solution was added to each well to stop the reaction, and the absorbance was measured at 450 nm using an ELISA reader to calculate the concentration of cortisol from the standard curve.

As a result, as shown in FIGS. 4 and 5, it was confirmed that the levels of cortisol, which were significantly increased due to sleep deprivation, were significantly reduced when Ja W + Pe W was administered compared to Ja W or Pe W alone.

### [Example 5]

### Confirmation of mixing ratio of natural extract mixture

When mixing the *Perilla frutescens var. acuta kudo* extract and *Mentha piperita* extract, in order to determine an optimal mixing ratio, the efficacy of the *Perilla frutescens var. acuta kudo* extract and *Mentha piperita* extract according to of each mixing ratio was evaluated using a corticosterone-induced stress cell model.

The mixed µg/ml concentration ratio of a *Mentha piperita* hot water extract (Pe W) and a *Perilla frutescens var. acuta kudo* hot water extract (Ja W) obtained in [Example 1] was 1 : 1, 1 : 2, 1 : 3, 1 : 4, 1 : 5, 1 : 6, 1 : 7, 1 : 8, 1 : 9 or 2 : 1, 3 : 1, 4 : 1, 5 : 1, 6 : 1, 7 : 1, 8 : 1, 9 : 1 or 8 : 2, 7 : 3, 6 : 4, 5 : 5, 4 : 6, 3 : 7, and 2 : 8 and an extract mixture at each ratio was prepared. The effect of individual materials on alleviating or restoring stress (anti-stress effect) on a stress response (proliferation inhibition) caused by corticosterone in nerve cells (SH-SY5Y cells) was investigated. The concentration of corticosterone as a stress inducer was 500 µM, and the mixed extract of each ratio was treated. At 24 hours, the cell proliferation value (efficacy (%)) was investigated with a negative control and a control group treated only with corticosterone through MTT assay.

As a result, as shown in FIGS. 6 to 8, it was confirmed that when the µg/ml concentration ratio of Pe W : Ja W was 1 : 5, 3 : 1, or 4 : 6, the anti-stress effect significantly increased compared to the case where Pe W or Ja W alone was treated.

### [Industrial Applicability]

Using *Mentha piperita* extract and *Perilla frutescens var. acuta kudo* extract in combination significantly alleviates stress and reduces cortisol levels as compared to using *Mentha piperita* extract or *Perilla frutescens var. acuta kudo* extract by itself, and thus the natural extract mixture in which *Mentha piperita* extract and *Perilla frutescens var. acuta kudo* extract are mixed together according to the present invention can be effectively used as an anti-stress composition, and furthermore, as a composition for preventing, ameliorating, or treating stress-related diseases, or in a method for preventing or treating stress-related diseases.

## Claims

1. An anti-stress composition comprising at least one extract selected from a *Mentha piperita* extract and a *Perilla frutescens var. acuta kudo* extract.

2. The anti-stress composition of claim 1, wherein the extract is extracted from at least one part selected from the group consisting of leaves, stems, flowers, fruits, and whole plants of *Mentha piperita* or *Perilla frutescens var. acuta kudo.*

3. The anti-stress composition of claim 1, wherein the extract is extracted using water, an organic solvent, or a mixture thereof as a solvent.

4. The anti-stress composition of claim 1, wherein the *Mentha piperita* extract and the *Perilla frutescens var. acuta kudo* extract are mixed at a µg/ml concentration ratio of 1 : 10 to 10 : 1.

5. The anti-stress composition of claim 1, wherein the composition is a fragrance composition, a cosmetic composition, or a quasi-drug composition.

6. A pharmaceutical composition for preventing or treating stress-related diseases comprising at least one extract selected from the group consisting of a *Mentha piperita* extract and a *Perilla frutescens var. acuta kudo* extract.

7. The pharmaceutical composition of claim 6, wherein the stress-related disease is at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

8. A health functional food composition for preventing or ameliorating stress-related diseases comprising at least one extract selected from the group consisting of a *Mentha piperita* extract and a *Perilla frutescens var. acuta kudo* extract.

9. The health functional food composition of claim 8, wherein the stress-related disease is at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.

10. A method for preventing or treating stress-related diseases comprising administering to a patient with stress-related diseases at least one extract selected from the group consisting of a *Mentha piperita* extract and a *Perilla frutescens var. acuta kudo* extract.

11. The method for preventing or treating stress-related diseases of claim 10, wherein the stress-related disease is at least one selected from the group consisting of stress tension, stress anxiety, stress depression, fatigue syndrome, fear memory, mental panic attack, obsessive-compulsive disorder, post-traumatic stress disorder, schizophrenia, stress headaches, neurodegenerative diseases, eating disorders, anorexia nervosa, drug addiction, drug or alcohol withdrawal syndromes, and stress psychotic episodes.
